# EUROPÄISCHE PATENTANMELDUNG

(11) **EP 1 690 543 A1**
(43) Veröffentlichungstag der Anmeldung: **16.08.2006**
(21) Anmeldenummer: 05003179.8
(22) Anmeldetag: 15.02.2005
(51) Int. Cl.: A61K 31/567, A61P 15/18

(54) **Pharmazeutisches Präparat zur Kontrazeption**

(71) Anmelder: Schering AG, 13342 Berlin (DE)
(72) Erfinder: Gräser, Thomas, 99102 Windischholzhausen (DE); Claussen, Claus, 07743 Jena (DE)
(74) Vertreter: Cramer, Eva-Maria

(57) **Zusammenfassung**

Ein pharmazeutisches Präparat enthält als erste Wirkstoffkomponente 17α-Cyanomethyl-17-β-hydroxyestra-4,9-dien-3on (Dienogest) in einer täglichen Dosierung von 1.5 mg bis 2 mg und als zweite Wirkstoffkomponente 17α-Ethinylestradiol (Ethinylestradiol) in einer täglichen Dosierung von 0.015 mg bis 0.02 mg gemeinsam mit einem oder mehreren pharmazeutisch annehmbaren Trägern. Das pharmazeutische Präparat wird mindestens 21 Tage eines 28-tägigen Menstruationszyklus oral verabreicht. Ferner enthält das pharmazeutische Präparat 7 oder weniger wirkstofffreie Tagesdosiseinheiten.

## Beschreibung

Die Erfindung betrifft ein pharmazeutisches Präparat, welches 17α-Cyanomethyl-17-β-hydroxyestra-4,9-dien-3on (Dienogest) und 17α-Ethinylestradiol (Ethinylestradiol) enthält und die empfängnisverhütende Verwendung dieser Kombination.

### Allgemeiner Stand der Technik

Orale empfängnisverhütende Mittel, bestehend aus einer Gestagenkomponente und einer Östrogenkomponente, kamen erstmals in den frühen 60er Jahren auf den Markt. Drei wesentliche Eigenschaften charakterisieren das Profil der "Verhütungspille": kontrazeptive Sicherheit, eine sehr gute Zykluskontrolle sowie ein Minimum an Nebenwirkungen. Die kontrazeptive Sicherheit beruht in erster Linie auf der Gestagenkomponente. Diese bewirkt eine Hemmung der Gonadotropinfreisetzung in der Hypophyse sowie eine Inhibierung der Ovulation. Außerdem führt die periphere Wirkung des Gestagens auf das Endometrium zur verminderten Wahrscheinlichkeit für die Implantation der befruchteten Eizelle; auf die Zervix zur Sekretion von zähem Sekret, welches die Spermien in der Gebärmutter reduziert. Auch ist eine periphere Wirkung auf die Eileiter zu verzeichnen.
Die Östrogenkomponente verstärkt den anovulatorischen Effekt des Gestagens und ist verantwortlich für eine akzeptable Zykluskontrolle.
Seit Einführung hormoneller Kontrazeptiva ist die Forschung auf die Entwicklung von Präparaten gerichtet, die bei gleichbleibend guter kontrazeptiver Sicherheit und Zykluskontrolle unerwünschte Nebenwirkungen, wie beispielsweise arterielle und venöse Thrombosen und Beeinflussung des Kohlehydrat- und Fettstoffwechsels, reduziert. Derartige Nebenwirkungen sind vor allem von oralen Kontrazeptiva der ersten Generation bekannt, welche einen höheren Gehalt an Gestagen und Östrogen enthielten, als zur Empfängnisverhütung notwendig war.

WO 98/004269 offenbart u. a. die orale Verabreichung einer Kombination von 250 µg - 4 mg Dienogest und 10 µg - 20 µg Ethinylestradiol zur Empfängnisverhütung. Um die wesentliche Verringerung des pro Zyklus verabreichten gesamten empfängnisverhütenden Steroids zu erreichen, während eine gute Zykluskontrolle beibehalten wird, verabreicht man die niedrig dosierte Gestagen/Östrogen-Kombination für 23 bis 25 Tage eines 28-tägigen Menstruationszyklus. Jedoch werden in der Patentschrift keine Ergebnisse und Angaben offenbart, die belegen, dass die erfinderische Idee auch zum Erfolg führt.
WO 01/015701 beansprucht eine pharmazeutische Zusammensetzung auch zur oralen Verabreichung von 21 Tagen eines 28-tägigen Menstruationszyklus, die Drospirenon und Ethinylestradiol u. a. auch niedrig dosiert enthält, wobei Drospirenon in mikronisierter Form vorliegt. Besonderes Augenmerk wird hierbei auf eine schnelle Freisetzung der Steroide gelegt.
Aus der Fach- und Patentliteratur ist bekannt, dass steroidale Wirkstoffe durch das Cytochrom P 450 Enzymsystem metabolisiert werden, welches auch gleichzeitig für den Abbau einer Reihe von Arzneimitteln verantwortlich ist. Bestimmte Arzneimittel, wie Barbiturate, Antiepileptika und ausgewählte Virostatika, induzieren dieses Enzymsystem und vermindern die Wirkstoffkonzentration der Steroide im Blut. Um dies auszugleichen, müsste eigentlich der Gestagengehalt gesteigert oder zumindest beibehalten und nicht reduziert werden, welches jedoch vorbezeichnete Nebenwirkungen auslöst.

### Detaillierte Offenbarung der Erfindung

Der Erfindung liegt die Aufgabe zugrunde, eine pharmazeutische Zusammensetzung auf Basis von Dienogest und Ethinylestradiol aufzuzeigen, in welcher die steroidale Gesamtdosierung reduziert ist, gleichzeitig das Gleichgewichtes zwischen Dienogest und Ethinylestradiol erhalten bleibt, die kontrazeptive Wirksamkeit und einegute Zykluskontrolle realisiert und Nebenwirkungen, wie beispielsweise zusätzliches Absinken der Wirkstoffspiegel im Blut bei gleichzeitiger Einnahme weiterer Arzneimittel, ausgleicht.
Diese Aufgabe wird erfindungsgemäß durch ein pharmazeutisches Präparat, 1.5 mg bis 2.0 mg Dienogest und 0.015 mg - 0.020 mg Ethinylestradiol enthaltend, gelöst, wobei die orale Gabe für mindestens 21 Tage eines 28-tägigen Menstruationszyklus erfolgt.
Die Wirkstoffkombination Dienogest/Ethinylestradiol ist gemeinsam mit einem oder mehreren pharmazeutisch annehmbaren Trägern oder Arzneimittelträgern im pharmazeutischen Präparat eingebracht. Ausführungsformen sind pharmazeutische Präparate in Form von Tablettenzubereitungen, geeignet für eine orale Verabreichung, enthaltend 2 mg Dienogest und 0.020 mg Ethinylestradiol oder 2 mg Dienogest und 0.15 mg Ethinylestradiol bzw. 1.5 mg Dienogest und 0.15 mg Ethinylestradiol.
Im erfindungsgemäßen pharmazeutischen Präparat besitzt vorzugsweise das Dienogest eine schnelle in-vitro-Freisetzung) und eine verzögerte in-vitro-Freisetzung und das Ethinylestradiol eine konventionelle schnelle in-vitro-Freisetzung.
Der verzögert freigesetzte Anteil an Dienogest beträgt mindestens 5 %, vorzugsweise größer 30 % wie mit dem Dissolutionstest nach Ph.Eur. mittels Drehkörbchenapparatur unter Verwendung von 1000 ml Wasser mit 37 °C als Dissolutionmedium und einer Rührgeschwindigkeit von 50 U/Min bestimmt,
Die schnelle in-vitro-Freisetzung der Dosis an Ethinylestradiol und des Anteils an der Dosis von Dienogest erfolgt zu mindestens 75 % in maximal 45 Min, vorzugsweise zu 70 % in 30 Min wie mit vorbezeichneten Dissolutionstest bestimmt.
Eine Ausführungsform ist eine Filmtablette mit einem wirkstoffhaltigen Filmüberzug , deren Tablettenkern den Gehalt an Dienogest der zweiten Phase und der Filmüberzug den Gehaltes an Dienogest der ersten Phase und den Gesamtgehalt an Ethinylestradiol enthält.
Es wurde überraschenderweise gefunden, dass die teilweise verzögerte Auflösung (Freisetzung) des Dienogest aus den Tablettenzubereitungen die niedrige Dosierung der Wirkstoffkombination Dienogest/Ethinylestradiol im erfindungsgemäßen Bereich ermöglicht.

Es hat sich herausgestellt, dass die Wirkstoffkombination im erfindungsgemäßen pharmazeutischen Präparat neben der Empfängnisverhütung antiandrogene Eigenschaften besitzt und daher bei der Prophylaxe und Therapie von androgeninduzierten Störungen, insbesondere von Akne, verwendet werden kann.
Bei geeigneten Ausführungsformen des erfindungsgemäßen pharmazeutischen Präparates kann die Anzahl der Tagesdosiseinheiten 22, 23, 24 oder 25 Tagesdosiseinheiten betragen und die Anzahl der wirkstofffreien Tagesdosiseinheiten beträgt dann 6, 5, 4 oder 3 im 28-tägigen Menstruationszyklus.
Es kann die Kombination von 1.5 mg bis 2.0 mg Dienogest und 0.015 mg bis 0.020 mg Ethinylestradiol zur Herstellung eines pharmazeutischen Präparates bzw. zur Zubereitung einer pharmazeutischen Zusammensetzung erfindungsgemäß zur Hemmung der Ovulation verwendet werden.

Empfängnisverhütende Wirksamkeit von Formulierungen, die Dienogest und Ethinylestradiol enthalten

In einer randomisierten, offenen klinischen Studie wurden 40 Frauen im Alter zwischen 18 und 35 Jahren, die ihr schriftliches Einverständnis zur Studienteilnahme gegeben hatten, mit 2 verschiedenen Formulierung, die Dienogest und Ethinylestradiol enthielten, behandelt. Die erste Formulierung (A) entsprach einer Kombination von 2 mg Dienogest (nicht verzögert freigesetzt) und 0.02 mg Ethinylestradiol. Die zweite Formulierung (B) bestand aus einer Kombination aus 1.5 mg Dienogest (zu 50 % = 0.75 mg verzögert freigesetzt) und 0.02 mg Ethinylestradiol.
Der Versuch umfasste einen Vorbehandlungszyklus (Wash-out-Phase), 3 Behandlungszyklen und einen Nachbehandlungszyklus (Follow-up-Phase).
Zu festgelegten Zeitpunkten (vor Beginn des ersten Behandlungszyklus, am Ende des dritten Behandlungszyklus) werden verschiedene laborchemische und funktionsdiagnostische Untersuchungen durchgeführt. Zum Nachweis der ovulationshemmenden Wirkung wurden FSH, LH, Estradiol, Progesteron, "Spinnbarkeit" und Farnkrautphänomen untersucht. Die Follikelreifung wurde mit Hilfe einer Ultraschalluntersuchung geprüft. Außerdem wurden SHBG, CBG, Gesamttestosteron, Triglyceride, Cholesterin, HDL, LDL, Glukose im Serum bestimmt sowie Blutdruck, Herzfrequenz, Körpergewicht und Blutungsverhalten registriert.
Die Ergebnisse der Studie belegen, dass beide Formulierungen die Ovulation sicher hemmen, was durch den Abfall von LH, FSH, Progesteron und Estradiol während der Behandlungszyklen belegt wird. In beiden Gruppen konnte durch die begleitende Ultraschalluntersuchung ein Follikelwachstum nachgewiesen werden, dass aber bei keiner Studienteilnehmerin zur Follikelruptur und damit zur Ovulation (Eisprung) führte. Bei 3 Frauen, die mit der Formulierung A behandelt wurden, konnten im Behandlungszyklus 2 und 3 so genannte LUFs (luteinized unruptured follicles) nachgewiesen werden, die einen Hinweis für stattgefundene Follikelreifung aber ausgebliebene Ovulation darstellen. SHBG und CB stiegen in beiden Behandlungsgruppen in vergleichbarer Weise an. Die hauptsächlich durch die Gestagenkomponente der Präparate beeinflussten Parameter "Spinnbarkeit" und Farnkrautphänomen waren in der mit der Formulierung B behandelten Gruppe tendenziell stärker vermindert als in der Vergleichsgruppe. Der Unterschied erreichte jedoch keine statistische Signifikanz. Der Anstieg von HDL-Cholesterol und der Abfall von LDL-Cholesterol war in der Behandlungsgruppe B signifikant stärker ausgeprägt als in der Gruppe A. Triglyceride und Gesamcholesterol zeigten in beiden Gruppen vergleichbare Veränderungen. Die Glukosetoleranz blieb in beiden Gruppen weitgehend unbeeinflusst. Gesamttestosteron nahm in vergleichbarer Weise und signifikant in beiden Behandlungsgruppen ab. Die subjektive und objektive Verträglichkeit der Formulierung B wurde als günstiger eingeschätzt. Es traten weniger Zwischenblutungen, insbesondere im ersten Behandlungszyklus auf. Auch die Intensität der regulären Gestagenentzugsblutung (in der Pillenpause) war geringer. Außerdem berichteten Frauen, die mit der Formulierung B behandelt wurden, über weniger prämenstruelle Beschwerden (Kopfschmerzen, Bauchschmerzen). Hinsichtlich Blutdruck, Herzfrequenz und Körpergewicht wurden keine Unterschiede zwischen den Gruppen und im zeitlichen Verlauf gefunden.
Die durchgeführte Studie hat gezeigt, dass beide Präparate den Eisprung bei allen Freiwilligen sicher verhindert haben. Es wurde eine Tendenz zu verstärkten, peripheren antikontrazeptiven Effekten unter der Formulierung B festgestellt. Einige ungünstige Nebenwirkungen (Blutungsunregelmäßigkeiten, prämenstruelle Beschwerden) traten unter der Formulierung B signifikant seltener auf. Für die Formulierung B wurde ein günstigeres Lipidprofil ermittelt.

Die Ergebnisse zeigen, dass durch eine verzögerte Auflösung (Freisetzung) von Dienogest eine niedrige Dosierung sowohl von Dienogest als auch von Ethinylestradiol ermöglicht wird (Formulierung B), d.h. die steroidale Gesamtdosierung reduziert ist und gleichzeitig dem Bedarf eines Gleichgewichtes zwischen Dienogest und Ethinylestradiol entsprochen wird. Ebenfalls wird eine gute kontrazeptive Wirksamkeit und Zykluskontrolle realisiert und derartige Nebenwirkungen, wie beispielsweise zusätzliches Absinken der Wirkstoffspiegel im Blut bei gleichzeitiger Einnahme weiterer Arzneimittel, ausgeglichen.
Auch die Formulierung A, welche eine Niedrigdosierung von Ethinylestradiol im Vergleich zu den konventionellen Präparaten beinhaltet, zeigt nachweisbaren ovulationshemmenden Effekt, jedoch nicht so umfassend wie die sowohl im Dienogest-Gehalt als auch Ethinylestradiol-Gehalt niedriger dosierte Formulierung B.

## Patentansprüche

1. Pharmazeutisches Präparat, 1.5 mg bis 2.0 mg Dienogest und 0.015 mg bis 0.020 mg Ethinylestradiol enthaltend, gemeinsam mit einem oder mehreren pharmazeutisch annehmbaren Trägern, wobei die orale Gabe für mindestens 21 Tage eines 28-tägigen Menstruationszyklus erfolgt.

2. Präparat nach Anspruch 1, wobei ein Anteil des Dienogest verzögert freigesetzt wird.

3. Präparat nach einem der Ansprüche 1 bis 2, wobei der verzögert freigesetzte Anteil an Dienogest mindestens 5 % beträgt wie mit dem Dissolutionstest unter Verwendung von Wasser mit 37 °C als Dissolutionmedium und 50 U/Min als Rührgeschwindigkeit bestimmt.

4. Präparat nach einem der Ansprüche 1 bis 3, wobei die Anzahl der Tagesdosiseinheiten, die die Kombination aus Dienogest und Ethinylestradiol enthalten, 22, 23, 24 oder 25 beträgt und die Anzahl der Tagesdosiseinheiten, die keinen Wirkstoff enthalten, 6, 5, 4 oder 3 beträgt.

5. Verwendung von 1.5 mg bis 2.0 mg Dienogest in Kombination mit 0.015 mg - 0.020 mg Ethinylestradiol zur Zubereitung einer pharmazeutischen Zusammensetzung zur Hemmung der Ovulation für 21 Tage eines 28-tägigen Menstruationszyklus.

6. Verwendung nach Anspruch 6, wobei ein Anteil des Dienogest verzögert freigesetzt wird.

7. Verwendung nach einem der Ansprüche 6 bis 7, wobei der verzögert freigesetzte Anteil an Dienogest mindestens 5 % beträgt wie mit dem Dissolutionstest unter Verwendung von Wasser mit 37 °C als Dissolutionmedium und 50 U/Min als Rührgeschwindigkeit bestimmt.

## Geänderte Patentansprüche

### Geänderte Patentansprüche gemäss Regel 86(2) EPÜ.

**1.** Pharmazeutisches Präparat zur oralen Kontrazeption, 1.5 mg Dienogest und 0.015 oder 0.020 mg Ethinylestradiol oder 2.0 mg Dienogest und 0.015 mg Ethinylestradiol enthaltend, gemeinsam mit einem oder mehreren pharmazeutisch annehmbaren Trägern zu mindestens 21 Tagesdosiseinheiten eines 28-tägigen Menstruationszyklus.

**2.** Präparat nach Anspruch 1, wobei das pharmazeutische Präparat eine Filmtablette ist, bestehend aus einem Tablettenkern mit einem Gehalt an Dienogest und einem wirkstoffhaltigen Filmüberzug mit einem Gehalt an Dienogest und einem Gesamtgehalt an Ethinylestradiol.

**3.** Präparat nach einem der Ansprüche 1 bis 2, wobei der Anteil an Dienogest aus dem Tablettenkern zu mindestens 30 %, vorzugsweise 50 % an Dienogest verzögert nach größer 30 Min und der Anteil an Dienogest ebenso wie der Gesamtgehalt an Ethinylestradiol aus dem Filmüberzug zu mindestens 75 % in maximal 45 Min, vorzugsweise zu 70 % in 30 Min heraus gelöst werden, wie mit dem Dissolutionstest unter Verwendung von Wasser mit 37 °C als Dissolutionmedium und 50 U/Min als Rührgeschwindigkeit bestimmt.

**4.** Präparat nach einem der Ansprüche 1 bis 3. wobei die Anzahl der Tagesdosiseinheiten, die die Kombination aus Dienogest und Ethinylestradiol enthalten, 22, 23, 24 oder 25 beträgt und die Anzahl der Tagesdosiseinheiten, die keinen Wirkstoff enthalten, 6, 5, 4 oder 3 beträgt.
